(19) Europäisches Patentamt
European Patent Office
Office européen des brevets

(11) Veröffentlichungsnummer: **0 469 523 B1**

# (12) EUROPÄISCHE PATENTSCHRIFT

(45) Veröffentlichungstag der Patentschrift: **19.04.95**

(21) Anmeldenummer: **91112739.7**

(22) Anmeldetag: **29.07.91**

(51) Int. Cl.[6]: **C12N 15/53**, C12N 9/04, C12N 1/20, C12Q 1/32, //(C12N1/20,C12R1:19)

## (54) Klonierung und Überexpression von Glucose-6-Phosphat-Dehydrogenase aus Leuconostoc dextranicus.

(30) Priorität: **30.07.90 DE 4024158**

(43) Veröffentlichungstag der Anmeldung:
**05.02.92 Patentblatt 92/06**

(45) Bekanntmachung des Hinweises auf die Patenterteilung:
**19.04.95 Patentblatt 95/16**

(84) Benannte Vertragsstaaten:
**AT BE CH DE DK ES FR GB GR IT LI LU NL SE**

(56) Entgegenhaltungen:

**FEBS LETTERS. Bd. 211, Nr. 2, Januar 1987, AMSTERDAM NL Seiten 243 - 246;BHADBHADE, M.M. ET AL.: 'Sequence identity between a lysine-containing peptidefrom Leuconostoc mesenteroides glucose-6-phosphate dehydrogenase and an activesite peptide from human erythrocyte glucose-6-phosphate dehydrogenase'**

(73) Patentinhaber: **BOEHRINGER MANNHEIM GMBH**

**D-68298 Mannheim (DE)**

(72) Erfinder: **Jarsch, Michael, Dr.**
**Unterkarpfensee 11**
**W-8173 Bad Heilbrunn (DE)**
Erfinder: **Lang, Gunter**
**Langestrasse 22**
**W-8132 Tutzing (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**EP 0 469 523 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF BACTERIOLOGY Bd. 169, Nr. 1, Januar 1987, AMERICAN SOCIETY FOR MICROBIOLOGY Seiten 334-339; MURPHY, n.b. et al.: "Expression of the gene for NAD-dependent glucose-6-phosphate dehydrogenase from Leuconostoc mesenteroidescloned in Escherichia coli K-12"

BIOCHEMICAL SOCIETY TRANSACTIONS Bd. 17, Nr. 2, April 1989, THE BIOCHEMICALSOCIETY; LONDON, GB Seiten 313 - 315; LEVY, R.H.: 'Glucose-6-phosphatedehydrogenase from Leuconostoc mesenteroides'

JOURNAL OF BIOLOGICAL CHEMISTRY. Bd. 266, Nr. 20, 15. Juli 1991, BALTIMORE USSeiten 13028 - 13034; LEE, T.W. ET AL.: 'Cloning of the gene and amino acidsequence for glucose 6-phosphate dehydrogenase from Leuconostoc mesenteroides'

## Beschreibung

Die Glucose-6-Phosphat-Dehydrogenase (G6P-DH) katalysiert den ersten Schritt im oxidativen Glucose-Metabolismus. Dabei wird Glucose-6-Phosphat zu Gluconsäure-6-Phosphat oxidiert, während $NAD^+$ oder/und $NADP^+$ als Cosubstrat reduziert werden. Durch die Glucose-Oxidation werden schließlich Pentosezucker für den Nucleinsäure-Stoffwechsel erzeugt.

Die Glucose-6-Phosphat-Dehydrogenase kann beispielsweise aus Leuconostoc mesenteroides gewonnen werden. Dieses Enzym kann sowohl $NAD^+$ als auch $NADP^+$ als Cofaktor verwenden, im Gegensatz zum Enzym aus Hefe, das für $NADP^+$ spezifisch ist. Das Enzym liegt als Dimer, bestehend aus zwei gleichen monomeren Untereinheiten mit einem Molekulargewicht von 55.000 D vor. Es besitzt bei 25 °C eine spezifische Aktivität von 550 U/mg.

Nachteile des Verfahrens zur Gewinnung von G6P-DH aus Bakterien der Gattung Leuconostoc bestehen u.a. darin, daß die Milchsäure-Bakterien komplexe Nährstoffansprüche besitzen und daher in den großtechnisch verwendeten Nährmedien nur langsam wachsen und eine geringe Zelldichte erreichen. Zudem ist der Gehalt der Biomasse an G6P-DH bei Leuconostoc nur sehr gering (ungefähr 1 % des Gesamt-Zellproteins). Zur Bereitstellung ausreichender Mengen an G6P-DH sind daher große Fermentations-Dimensionen notwendig. Weiterhin kann wegen der großen Mengen an Fremdprotein nur eine Enzympräparation mit geringer spezifischer Aktivität erhalten werden.

Der bedeutendste Nachteil der bekannten G6P-DH aus Leuconostoc-Bakterien besteht jedoch in ihrer geringen Temperaturstabilität.

Aufgabe der vorliegenden Erfindung war es somit, eine Glucose-6-Phosphat-Dehydrogenase bereitzustellen, welche die Nachteile des Standes der Technik nicht mehr aufweist.

Die erfindungsgemäße Aufgabe wird durch Bereitstellung einer Glucose-6-Phosphat-Dehydrogenase gelöst, welche die in SEQ ID NO:1 dargestellte Aminosäuresequenz enthält und aus Leuconostoc mesenteroides, Subspezies dextranicus (DSM 20187), im weiteren als Leuconostoc dextranicus bezeichnet, erhältlich ist.

Weiterhin ein Gegenstand der vorliegenden Erfindung ist auch eine DNA, welche die in SEQ ID NO:1 dargestellte oder eine ihr im Rahmen der Degeneration des genetischen Codes entsprechende erfindungsgemäße Enzym kodierende Sequenz enthält.

Die erfindungsgemäße rekombinante DNA wurde durch Mustern einer L. dextranicus (DSM 20187) Genbank mit einer geeigneten Oligonukleotid-Sonde isoliert, wie weiter unten ausführlicher beschrieben ist.

Bei Expression der erfindungsgemäßen rekombinanten DNA in E. coli Zellen zeigte sich überraschenderweise, daß bereits geringe Fermentations-Volumina zur Bereitstellung der gewünschten Enzymmenge ausreichen. Im Vergleich zur Gewinnung der G6P-DH aus Leuconostoc wird eine Verringerung des Fermentations-Volumens um den Faktor 1:500 bis 1:1000 erreicht. Überdies erhält man bei verringertem Aufreinigungsaufwand G6P-DH-Präparationen von hoher Reinheit, d.h. mit einer spezifischen Aktivität von ca. 900 U/mg. Insbesondere zeichnet sich jedoch das erfindungsgemäße rekombinante Enzym bei Isolierung aus E. coli überraschenderweise durch eine deutlich verbesserte Temperaturstabilität gegenüber dem bekannten Enzym aus. Ein zusätzlicher Vorteil des rekombinanten Enzyms ist, daß es überraschenderweise im Gegensatz zum bekannten Enzym aus Leuconostoc nicht mit Glucose reagiert. Diese bekannte unspezifische Reaktion des Leuconostoc-Enzyms mit Glucose (Olive und Levy, Biochemistry 6 (1967), 730) war bislang ein erheblicher Nachteil bei der Durchführung von Enzymtests, da somit aufgrund des Vorhandenseins von Glucose in Blut, Serum oder Plasma falsche Ergebnisse bei Bestimmungen auftreten konnten. Schließlich unterscheidet sich das rekombinante Enzym von der bekannten G6P-DH auch durch einen unterschiedlichen $K_m$-Wert für $NADP^+$ und eine unterschiedliche Wirkung von Aktivatoren und Inhibitoren (z.B. Phosphat, Glycerin, Magnesiumionen, Hydrogencarbonat).

Weiterhin ein Gegenstand der vorliegenden Erfindung ist ein rekombinanter Vektor, der eine oder mehrere Kopien der erfindungsgemäßen rekombinanten DNA enthält. Ein derartiger Vektor soll die Expression der erfindungsgemäßen rekombinanten DNA in fremden Wirtsorganismen ermöglichen. Der erfindungsgemäße Vektor kann ein Vektor sein, der sich in die chromosomale DNA der Wirtszelle integriert (z.B. Bakteriophage Lambda), er kann jedoch auch in der Wirtszelle extrachromosomal vorliegen (Plasmid). Vorzugsweise ist der erfindungsgemäße Vektor ein Plasmid.

Der erfindungsgemäße Vektor kann sowohl ein eukaryontischer als auch ein prokaryontischer Vektor sein, vorzugsweise ist er jedoch ein prokaryontischer Vektor, d.h. zur Vermehrung in prokaryontischen Wirtsorganismen geeignet. Besonders bevorzugt besitzt der rekombinante Vektor einen in E. coli aktiven Replikationsursprung, d.h. er ist in E. coli vermehrbar.

In einer besonders bevorzugten Ausführungsform enthält der erfindungsgemäße rekombinante Vektor die für die Glucose-6-Phosphat-Dehydrogenase kodierende Nukleinsäuresequenz unter Kontrolle einer in E.coli funktionsfähigen Promotorsequenz aus Leuconostoc dextranicus, die in den ersten 122 Nukleotiden (stromaufwärts des G6P-DH-Gens) der in SEQ ID NO:1 dargestellten Nukleinsäuresequenz enthalten ist.

Um Promotoreigenschaften aufzuweisen ist es nicht erforderlich, daß der DNA-Bereich genau diese Sequenz von 122 Nukleotiden besitzt. Auch abgeleitete Sequenzen oder Fragmente dieser Frequenz, die Promotoreigenschaften besitzen, sind geeignet. Unter einer abgeleiteten biologisch aktiven Sequenz im Sinne der Erfindung versteht man daher, daß einzelne Nukleotide oder kurze Nukleotid-Sequenzen aus der Promotorsequenz deletiert, substituiert oder insertiert sein können, und zwar auf solche Weise, daß die Promotor-Aktivität der Sequenz erhalten bleibt. Dem Fachmann ist ja bekannt, daß bei einem Promotor nicht die gesamte Sequenz, sondern nur bestimmte Teilbereiche konserviert bleiben müssen. Bei prokaryontischen Promotorsequenzen sind dies insbesondere die Bereiche bei -35 und bei -10 bezüglich des Transkriptionsstarts.

Die Erfindung beinhaltet somit auch eine rekombinante DNA, welche die ersten 122 Nukleotide der in SEQ ID NO:1 dargestellten Nukleinsäure-Sequenz oder eine davon abgeleitete Sequenz mit Promotoreigenschaften besitzt. Überraschenderweise ergibt dieser Leuconostoc Promotor auch in E. coli eine gute Proteinexpression. Somit kann man diesen Promotor auch zur Expression heterologer Gene, d.h. vom G6P-DH-Gen verschiedenen Genen in gram-negativen Bakterien, vorzugsweise E. coli Bakterien verwenden.

Weiterhin ein Gegenstand der vorliegenden Erfindung ist ein Mikroorganismus, der mit einem erfindungsgemäßen rekombinanten Vektor transformiert ist. Vorzugsweise handelt es sich dabei um ein gramnegatives Bakterium, besonders bevorzugt um ein E. coli Bakterium.

Die erfindungsgemäße rekombinante DNA kann man erhalten, indem man

(1) chromosomale Leuconostoc dextranicus DNA isoliert und mit einem geeigneten Restriktionsenzym spaltet,

(2) die gespaltene L. dextranicus DNA in einen Vektor einbaut, mit dem Vektor einen geeigneten Organismus transformiert und auf diese Weise eine Genbank herstellt,

(3) die Genbank aus Schritt (2) mit einer Nukleinsäure-Sonde mustert, die eine für das Glucose-6-Phosphat-Dehydrogenase-Gen spezifische Sequenz besitzt, wobei man diese Sonden im Hinblick auf die Codon-Verwertung in Milchsäurebakterien konstruiert, und

(4) die mit der Sonde aus Schritt (3) positiv reagierenden Klone der Genbank analysiert.

Die Isolierung von chromosomaler L. dextranicus (DSM 20187) DNA ist durch kombinierte Polyethylenglykol/Lysozym-Behandlung und anschließende Inkubation mit Proteinase K möglich.

Die Spaltung der isolierten L. dextranicus DNA mit einem geeigneten Restriktionsenzym, die Ligierung der gespaltenen DNA in einen geeigneten Klonierungsvektor, die Transformation eines geeigneten Organismus mit dem rekombinanten Klonierungsvektor zur Herstellung einer Genbank kann auf eine dem Fachmann auf dem Gebiet der Molekularbiologie an sich bekannte Weise erfolgen. Der nächste Schritt ist das Mustern der auf diese Weise hergestellten Genbank mit einer Nukleinsäure-Sonde, die eine für das Glucose-6-Phosphat-Dehydrogenase-Gen spezifische Sequenz besitzt.

Aus Haghighi et al., Biochemistry 21 (1982), 6415-6420 ist eine Peptidsequenz der G6P-DH aus L. mesenteroides mit einem pyridoxylierbaren Lysinrest (*) bekannt. Diese Sequenz lautet: Phe-Leu-Leu-Lys*-Ser-Pro-Ser-Tyr-(Asp/Val)-Lys. Aus dieser Sequenz konnte jedoch keine Oligonukleotid-Sonde abgeleitet werden, mit der man in der L. dextranicus Genbank ein Hybridisierungssignal finden kann.

Aus Bhadbhade et al., FEBS Letters 211 (1987), 243-246 ist eine Peptidsequenz aus dem aktiven Zentrum der G6P-DH aus L. mesenteroides mit einer hohen Homologie zur menschlichen G6P-DH bekannt. Aus der Vielzahl von daraus konstruierbaren Oligonukleotid-Sonden wurde die in Beispiel 2 genannte Oligonukleotid-Sonde mit einer Länge von 72 Basen (SEQ ID NO:2) hergestellt.

Ein Mustern der L. dextranicus DNA-Genbank mit diesem Oligonukleotid in 5'-endmarkierter Form erbrachte schließlich einen positiven Klon, durch den die Sequenz des L. dextranicus G6P-DH-Gens ermittelt werden konnte.

Die DNA-Sequenz des G6P-DH-Gens aus L. dextranicus wurde nach dem Verfahren von Sanger bestimmt. Sie ist in SEQ ID NO:1 dargestellt.

SEQ ID NO:1 zeigt ebenfalls die daraus ermittelte Aminosäuresequenz der G6P-DH aus L. dextranicus. Daraus ist ersichtlich, daß die Aminosäuresequenz des erfindungsgemäßen Enzyms in 6 von 42 Positionen nicht mit der in FEBS Letters 211 (1987), 243-246 beschriebenen Sequenz des L. mesenteroides-Enzyms übereinstimmt.

Weiterhin beinhaltet die Erfindung ein Verfahren zur Gewinnung einer G6P-DH mit der in SEQ ID NO:1 dargestellten Aminosäuresequenz, indem man

(1) einen geeigneten Wirtsoorganismus mit einer erfindungsgemäßen DNA oder einem Vektor transformiert, der eine oder mehrere Kopien dieser DNA enthält,

(2) den transformierten Wirtsorganismus in einem geeigneten Medium kultiviert und

(3) das Protein aus dem Medium oder den Zellen anreichert.

Die Expression des erfindungsgemäßen rekombinanten Proteins in einem transformierten Wirtsorganismus, vorzugsweise in einem prokaryontischen Wirtsorganismus, besonders bevorzugt in einer E. coli-Zelle, ist prinzipiell unter Kontrolle jedes geeigneten Promotors möglich. So ist in E. coli z.B. eine Expression der G6P-DH unter Kontrolle von heterologen Promotoren wie z.B. dem tac-Promotor, mgl-Promotor oder pfl-Promotor möglich. Vorzugsweise erfolgt jedoch die Expression konstitutiv unter Kontrolle eines Leuconostoc-Promotors, besonders bevorzugt unter Kontrolle der in SEQ ID NO:1 dargestellten oder einer davon abgeleiteten Promotor-Sequenz (entsprechend den ersten 122 Nukleotiden von SEQ ID NO:1). Am meisten bevorzugt ist das Plasmid pUC-G6P-DH1.8, das in Abb. 1 dargestellt ist.

Als geeigneter E. coli Wirtsstamm wurde der kommerziell erhältliche E. coli Stamm HB 101 ausgewählt. Bei Transformation von E. coli HB101 mit pUC-G6P-DH1.8 wurde gefunden, daß das Plasmid eine hohe Stabilität in der Zelle besitzt und die Expression der G6P-DH über mehrere Passagen auch ohne Selektionsdruck durchgeführt werden kann.

Die vorliegende Erfindung soll durch die folgenden Beispiele in Verbindung mit SEQ ID NO:1 und 2 sowie der Abbildung 1 weiter verdeutlicht werden. Es zeigen:

| | |
|---|---|
| SEQ ID NO:1 | die Nukleotidsequenz der Leuconostoc DNA-Insertion in pUC-G6P-DH1.8, wobei die ersten 122 Basen stromaufwärts des kodierenden Bereichs den L. dextranicus G6P-DH Promotor und die Basen 123-1580 die Nukleotidsequenz des L. dextranicus G6P-DH Gens darstellen, das für ein Protein mit der ebenfalls angegebenen Amminosäuresequenz kodiert, |
| SEQ ID NO:2 | die Oligonukleotidsonde für den Teil des G6P-DH Gens aus Leuconostoc mesenteroides, der für einen Bereich des aktiven Zentrums der G6P-DH von L. mesenteroides codiert, der eine hohe Homologie mit der menschlichen G6P-DH aufweist. |
| Abb. 1 | das Plasmid pUC-G6P-DH1.8, |

**Beispiel 1**

Isolierung chromosomaler DNA aus Leuconostoc dextranicus

Genomische DNA von Leuconostoc dextranicus wird nach folgendem Verfahren gewonnen:
Leuconostoc dextranicus (DSM 20187) wird in APT-Medium (Merck Nr. 10454) bei 30 °C angezogen. Die Zellen aus 100 ml Kulturbrühe werden abzentrifugiert, in 10 ml 20 mmol/l Tris/HCl pH 8,0 gewaschen und schließlich in 15 ml dieser Pufferlösung resuspendiert. Nach Zugabe von 5 ml 24 % (w/v) Polyethylenglykol 6000 und 20 mg Lysozym wird 16 h bei 4 °C inkubiert. Die Zellyse erfolgt durch Zugabe von 1 ml 20 % (w/v) SDS. Es werden 2 mg Protease K zugegeben und 60 min bei 37 °C inkubiert. Die weitere Reinigung der DNA erfolgt durch sequentielle Phenol- und Chloroform-Extraktion, Behandlung mit RNAse A (0,5 mg/60 min bei 37 °C), nochmaliger Phenol- und Chloroform-Extraktion und abschließender Ethanolpräzipitation.

**Beispiel 2:**

Bestimmung der Größe von genomischen DNA-Fragmenten, die für G6P-DH kodieren

Zur Hybridisierung wurde das in SEQ ID NO:2 gezeigte Oligonukleotid verwendet.

Je 5 µg genomischer DNA aus L. dextranicus wird mit verschiedenen Restriktionsendonukleasen (BclI, ClaI, HindIII, PstI, XbaI) gespalten, auf einem 0,8 % Agarosegel elekrophoretisch aufgetrennt und danach auf Nitrozellulosefilter transferiert. Ein solcher Filter wird nach Vorhybridisierung mit einer Lösung aus 6 x SSC-Puffer, 0,7 % Magermilchpulver, bei 40 °C über Nacht in der gleichen Lösung inkubiert, die zusätzlich das obige Oligonukleotid, mit $^{32}P$ radioaktiv endmarkiert, enthält. Nach Waschen, Trocknen und Autoradiographie kann festgestellt werden, daß ein durch das Restriktionsenzym BclI erzeugtes DNA-Fragment von ca. 3,4 kb Größe mit dem Oligonukleotid hybridisiert.

**Beispiel 3:**

Klonierung eines DNA-Fragments, das für G6P-DH kodiert

20 µg genomischer DNA aus L. dextranicus wird mit BclI geschnitten und in einem Gel aus niedrigschmelzender Agarose aufgetrennt. DNA-Fragmente mit einer Größe von ca. 3,4 kb +/-0,2 kb werden aus dem Gel ausgeschnitten. Dieses Gelstück wird mit Ligasepuffer (Maniatis et al. 1982, Molecular Cloning, p.

474) equilibriert und bei 65°C verflüssigt. Danach wird 0,1 µg pUC18 DNA, BamHI-gespalten und 5 U T4-Ligase zugegeben, 10 min bei 37°C, dann 16 h bei 15°C inkubiert. Die Restriktionsendonuklease BamHI erzeugt überhängende DNA-Enden, die zu den von BclI erzeugten Enden kompatibel sind.

Zellen von E. coli HB101 (DSM 1607) werden in 20 ml Nährmedium angezogen und durch Calciumchloridbehandlung in kompetenten Zustand überführt (Maniatis et al. 1982, Molecular Cloning, pp. 250 - 252). Der oben erhaltene Ligationsansatz wird nach Zugabe von einem Volumenteil 50 mmol/l Tris/HCl pH 7,5 nochmals 5 min bei 65°C verflüssigt und zur Transformation eingesetzt. Die so behandelten Zellen werden auf LB-Agarplatten mit 50 µg/ml Ampicillin ausplattiert und bei 37°C einen Tag bebrütet.

Die hochgewachsenen Kolonien werden auf neue LB-Agarplatten mit 50 µl Ampicillin überstempelt, auf denen Nitrozellulosefilter liegen. Nachdem die Kolonien abermals hochgewachsen sind, werden die Filter abgehoben, die Kolonien wie bei Grunstein und Hogness Proc. Natl. Acad. Sci. USA, 72 (1975) 3961 beschrieben, lysiert und mit der unter 2. beschriebenen radioaktiv markierten Oligonukleotidsonde hybridisiert. Nach Autoradiographie können Klone mit rekombinanten, G6P-DH-kodierenden Plasmiden identifiziert und von den Ausgangsplatten isoliert werden. Nach Isolierung und Charakterisierung der Plasmid-DNA solcher Klone zeigt sich, daß diese eine Größe von ca. 6 kb besitzen. Dies bedeutet, daß ein DNA-Fragment von ca. 3,4 kb Größe in die pUC18-DNA insertiert ist. Ein solches rekombinantes Plasmid wird für die weitere Bearbeitung ausgewählt.

**Beispiel 4:**

Einengung und Expression des Gens

Durch Spaltung mit den Restriktionsenzymen XbaI und SpeI kann das oben erhaltene rekombinante Plasmid in ein Fragment von ca. 2,2 kb und eines von ca. 3,8 kb Größe zerlegt werden. Dieses 3,8 kb-Fragment enthält nur noch DNA-Sequenzen aus pUC18 und die Nucleotidsequenz von SEQ ID NO:1. Isolierung und Religation des 3,8 kb Fragments und nachfolgende Transformation in E. coli HB101 führt zu einem Klon, der das G6P-DH-Gen exprimiert. Das G6P-DH Gen ist in diesem positiven Klon als 1,8 kb Fragment (SpeI/KpnI) in einem, im Polylinkerbereich mit XbaI und KpnI geschnittenen kommerziellen pUC18 Vektor subkloniert, wobei die KpnI-Schnittstelle aus dem Vektoranteil des positiven Klons aus der Genbank stammt. Damit ist ein SpeI/BclI-Fragment aus dem Leuconostoc dextranicus Genom vorhanden. Die DNA-Sequenz des gesamten subklonierten 1,8 kb SpeI/BclI Fragments ist in SEQ ID NO:1 dargestellt.

Das resultierende rekombinante Plasmid enthält das G6P-DH Gen unter Kontrolle des eigenen Leuconostoc-Promotors. Es wurde als pUC-G6P-DH1.8 bezeichnet und ist in Abb. 1 dargestellt.

Die Expressionsrichtung des G6P-DH Gens liegt dabei in Gegenrichtung zum lac-Promotor (pLAC) auf pUC18.

Zur Bestimmung der Enzymaktivität und Aufreinigung der G6P-DH wird ein solcher Klon in ein Reagenzglas mit 5 ml LB-Nährmedium mit 50 µg/ml Ampicillin eingeimpft und über Nacht bei 37°C angezogen. Mit dieser Kultur wird ein Erlenmeyerkolben mit 1 l LB-Nährmedium mit 50 µg/ml Ampicillin beimpft und nochmals über Nacht bei 37°C unter Schütteln bebrütet. Die Zellen werden durch Zentrifugation geerntet.

**Beispiel 5**

Anreicherung und Charakterisierung von rekombinanter G6P-DH aus E. coli

5.1 Anreicherungsprozedur

1. Aufschluß

5 kg Biomasse (E. coli HB101 pUC-G6P-DH1.8) mit 25 l Kaliumphosphatpuffer 10 mmol/l, pH 7,5 enthaltend $10^{-3}$ mol/l $MgCl_2$ suspendieren und die Zellen mit einem APV-Gaulin Hochdruckhomogenisator bei 800 bar Homogenisierdruck aufschließen. Die resultierende Suspension auf +4°C kühlen und zentrifugieren.

2. Ammoniumsulfat-Fraktionierung

Den Rohextrakt mit festem Ammoniumsulfat bis zu einer Konzentration von 1,9 mol/l versetzen und den ausgefallenen Niederschlag abzentrifugieren. Den Überstand weiter mit Ammoniumsulfat bis zu einer Konzentration von 3,0 mol/l ausfällen und den Niederschlag abzentrifugieren.

3. Erwärmung

Den zweiten Ammonsulfat-Niederschlag mit 20 mmol/l Kaliumphosphatpuffer, pH 6,0, enthaltend 1 mmol/l EDTA auflösen und 20 min auf 52 °C erwärmen. Den ausgefallenen Niederschlag abzentrifugieren.
4. Erste Kristallisation
Den Überstand von 3. langsam mit festem Ammoniumsulfat bis 2,1 mol/l versetzen; den pH-Wert mit NaOH auf 6,0 korrigieren. Über Nacht kristallisiert die G6P-DH aus. Die Kristallisation sollte bei Raumtemperatur und unter leichtem Rühren durchgeführt werden. Die Enzymkristalle abzentrifugieren.
5. Zweite Kristallisation
Den Niederschlag mit 20 mmol/l Kaliumphosphatpuffer, pH 6,0, enthaltend 1 mmol/l EDTA, lösen und mit festem Ammoniumsulfat bis 1,9 mol/l versetzen. Den pH-Wert wiederum auf 6,0 korrigieren und über Nacht bei Raumtemperatur und leichtem Rühren das Enzym auskristallisieren lassen.
6. Dialyse
Enzymkristallisat abzentrifugieren und den Niederschlag konzentriert mit 10 mmol/l Kaliumphosphatpuffer, pH 6,0, enthaltend 1 mmol/l EDTA lösen und 24 Stunden gegen gleichen Puffer dialysieren.
7. Lyophilisation
Die Enzymlösung ohne Zusätze lyophilisieren. Es resultieren ca. 210 g Lyophilisat mit ca. 900 U/mg Aktivität.

5.2 Charakterisierung von rekombinanter G6P-DH

Die gentechnologisch hergestellte G6P-DH unterscheidet sich in ihren Eigenschaften von dem bekannten Enzym aus Leuconostoc.

Die Nachteile des bekannten Leuconostoc-Enzyms sind wie folgt:
Die Langzeitstabilität ist gering. Weiterhin setzt das Enzym Glucose um, was bei Messungen in Blut, Serum oder Plasma zu falschen Ergebnissen führen kann, da in derartigen Proben immer Glucose enthalten ist. Die mangelnde Spezifität der G6P-DH ist in Archives of Biochemistry and Biophysics 149 (1972) 102-109 beschrieben.

Die Unterscheidungsmerkmale sind:
1. $K_m$ NADP (in 0,1 mol/l Tris pH 7,8; 25 °C

| rec G6P-DH | G6P-DH aus Leuconostoc |
|---|---|
| $3{,}7 \cdot 10^{-5}$ | $7{,}4 \cdot 10^{-6}$ mol/l [1]<br>$5{,}7 \cdot 10^{-6}$ mol/l [3]<br>$9{,}9 \cdot 10^{-6}$ mol/l [2] |

[1] Olive und Levy, Biochem. 6 (1967), 730
[2] DeMoss, in Methods in Enzymology, Vol 1., S. 328, Acad. Press, New York, 1955
[3] Levy, 626th Meeting Sheffield , S. 13 (1988)

2. Wirkung von Aktivatoren/Inhibitoren

| Zusatz von | rel. Aktivität (bezogen auf Aktivität ohne Zusatz) | | | |
|---|---|---|---|---|
| | rec. G6P-DH Cosubstrat | | G6P-DH aus Leuconostoc Cosubstrat | |
| | $NAD^+$ | $NADP^+$ | $NAD^+$ | $NADP^+$ |
| 5 mmol/l Phosphat | 100 % | 80 % | Aktivierung [2] | |
| 50 mmol/l Phosphat | 100 % | 80 % | 107 % [1] | 118 % [1] |
| 30 % Glycerin | 60 % | 30 % | 30 % [1] | 30 % [1] |
| 30 mmol/l $Mg^{2+}$ | 100 % | 100 % | 80 % [1] | 80 % [1] |
| 0,3 mol/l Hydrogencarbonat | 100 % | 100 % | 120 % [1] | 120 % [1] |

[1] Olive und Levy, Biochem. 6 (1967), 730
[2] DeMoss, in Methods in Enzymology, Vol 1., S. 328, Acad. Press, New York, 1955

3. Spezifität

| Spezifität für | rec G6P-DH mit $NAD(P)^+$ | G6P-DH aus Leuconostoc |
|---|---|---|
| Glucose[4] | kein Umsatz | Umsatz[5] |
| 2-Desoxyglucose-6P[4] | 5 % | kein Umsatz |

[4] Konzentration 0,15 mol/l
[5] Arch.Biochem. and Biophys. 149 (1972), 102-109

4. Temperaturstabilität

| Temperatur | rel. Aktivität rec. G6P-DH | (bezogen auf 20 °C) G6P-DH aus Leuconostoc dextranicus hergestellt nach [1] |
|---|---|---|
| 40 °C | 100 % | 100 % |
| 50 °C | 100 % | 97 % |
| 60 °C | 100 % | 90 % |
| 70 °C | 43 % | 4 % |

[1] Olive und Levy, Biochem. 6 (1967), 730

Die Bestimmung der Temperaturstabilität erfolgte in 3,2 mol/l Ammoniumsulfat pH 6,0 über 10 Minuten. (Anfangsaktivität des Enzyms 2500 U/ml).

Die Aktivitätsbestimmung und Spezifitätsbestimmung erfolgte wie in Beispiel 6 beschrieben.

## Beispiel 6

Bestimmung der Aktivität von Glucose-6-Phosphat-Dehydrogenase

G6P-DH setzt Glucose-6-Phosphat und $NAD^+$ in Gluconat-6-Phosphat und NADH um. Das gebildete NADH wird bei 340 nm photometrisch gemessen.

Zu 3 ml eines Reagenzes, bestehend aus Tris-Puffer (0,1 mol/l pH 7,8, 3 mmol/l $MgCl_2$), 0,1 mmol/l $NAD^+$, freie Säure und 0,15 mol/l Glucose-6-Phosphat werden 0,05 ml Probe (G6P-DH, Volumenaktivität möglichst zwischen 0,3 und 0,5 U/ml) bei 25 °C zugegeben und die Zunahme der Extinktion ($\Delta E$/min) verfolgt. Die Volumenaktivität errechnet sich nach:

$$\text{Volumenaktivität} = \frac{3{,}05}{\epsilon \times 1 \times 0{,}05} \times \Delta E\ \text{min}\ [\text{U/ml}]$$

$\epsilon_{340} = 6{,}3\ [\text{mmol}^{-1} \times \text{l} \times \text{cm}^{-1}]$

SEQ ID NO:1
ART DER SEQUENZ:Nucleotid mit entsprechendem Protein
SEQUENZLÄNGE:1696 Basenpaare
STRANGFORM:Einzelstrang

```
TCTAGTCATT TAATCAATTT TTGACTTGTT CAACGCTTAA TATGTTTGTG AATCCCGTAC   60

TTTTCCAGAC CTTTTTGCGT TATAATGGAG AGTGAATTTA ATTATAATAT AAGGGGAACA  120

TC                                                                 122

ATG GTT TCA GAA ATC AAA ACG TTG GTA ACT TTC TTT GGC GGA ACT GGT   170
Met Val Ser Glu Ile Lys Thr Leu Val Thr Phe Phe Gly Gly Thr Gly
                5                   10                  15

GAT TTA GCA AAG CGT AAG CTT TAC CCA TCA GTT TTC AAC CTC TAC AAA   218
Asp Leu Ala Lys Arg Lys Leu Tyr Pro Ser Val Phe Asn Leu Tyr Lys
            20                  25                  30

AAA GGA TAC TTA CAA GAA CAC TTT GCC ATT GTT GGG ACA GCA CGT CAA   266
Lys Gly Tyr Leu Gln Glu His Phe Ala Ile Val Gly Thr Ala Arg Gln
        35                  40                  45

CAA TTA AGT GAT GAC GAG TTT AAG CAA TTG GTT CGT GAT TCA ATT AAA   314
Gln Leu Ser Asp Asp Glu Phe Lys Gln Leu Val Arg Asp Ser Ile Lys
    50                  55                  60

GAC TTT ACT GAA GAT CAA GCA CAA GCC GAA GCG TTT ATT GCG CAT TTT   362
Asp Phe Thr Glu Asp Gln Ala Gln Ala Glu Ala Phe Ile Ala His Phe
65                  70                  75                  80

TCT TAC CGT GCG CAC GAT GTC ACA GAT GCC GCT TCT TAT GGT ATC TTG   410
Ser Tyr Arg Ala His Asp Val Thr Asp Ala Ala Ser Tyr Gly Ile Leu
                85                  90                  95

AAG TCA GCG ATC GAA GAA GCA GCA ACC AAA TTT GAC ATT GAT GGC AAT   458
Lys Ser Ala Ile Glu Glu Ala Ala Thr Lys Phe Asp Ile Asp Gly Asn
            100                 105                 110

CGT ATT TTC TAT ATG TCA GTT GCC CCT CGT TTC TTC GGT ACA ATC GCT   506
Arg Ile Phe Tyr Met Ser Val Ala Pro Arg Phe Phe Gly Thr Ile Ala
        115                 120                 125

AAA TAT TTG AAA TCA GAA GGT TTG CTA GCT GAG ACT GGC TAC AAT CGT   554
Lys Tyr Leu Lys Ser Glu Gly Leu Leu Ala Glu Thr Gly Tyr Asn Arg
    130                 135                 140

TTG ATG ATT GAA AAG CCT TTT GGT ACA TCA TAC GCC ACC GCA GAA GAA   602
Leu Met Ile Glu Lys Pro Phe Gly Thr Ser Tyr Ala Thr Ala Glu Glu
145                 150                 155                 160

TTG CAA AGT GAT TTG GAA AAT GCA TTT GAT GAT GAC CAA CTG TTC CGT   650
Leu Gln Ser Asp Leu Glu Asn Ala Phe Asp Asp Asp Gln Leu Phe Arg
                165                 170                 175
```

```
ATT GAC CAC TAT CTT GGA AAA GAA ATG GTA CAA AAT ATT GCA GCA TTA     698
Ile Asp His Tyr Leu Gly Lys Glu Met Val Gln Asn Ile Ala Ala Leu
            180                 185                 190

CGT TTT GGT AAC CCA ATC TTT GAT GCC GCT TGG AAT AAG GAC TAT ATC     746
Arg Phe Gly Asn Pro Ile Phe Asp Ala Ala Trp Asn Lys Asp Tyr Ile
        195                 200                 205

AAA AAC GTA CAA GTA ACT TTG GCT GAA GTT CTA GGT GTT GAA GAG CGT     794
Lys Asn Val Gln Val Thr Leu Ala Glu Val Leu Gly Val Glu Glu Arg
    210                 215                 220

GCT GGT TAC TAC GAT ACC ACT GGC GCC CTT TTG GAT ATG ATT CAA AAC     842
Ala Gly Tyr Tyr Asp Thr Thr Gly Ala Leu Leu Asp Met Ile Gln Asn
225                 230                 235                 240

CAC ACA ATG CAA ATT GTT GGT TGG TTA GCA ATG GAA AAA CCT GAA TCA     890
His Thr Met Gln Ile Val Gly Trp Leu Ala Met Glu Lys Pro Glu Ser
                245                 250                 255

TTC AAT GAT AAG GAT ATC CGT GCA GCT AAA AAC GCC GCC TTC AAT GCA     938
Phe Asn Asp Lys Asp Ile Arg Ala Ala Lys Asn Ala Ala Phe Asn Ala
            260                 265                 270

TTA AAG ATT TAT AAC GAA GAA GAA GTG AAT AAG TAC TTC GTT CGT GCA     986
Leu Lys Ile Tyr Asn Glu Glu Glu Val Asn Lys Tyr Phe Val Arg Ala
        275                 280                 285

CAA TAT GGT GCT GGT GAT ACA GCT GAT TAC AAG CCA TAT TTG GAA GAA    1034
Gln Tyr Gly Ala Gly Asp Thr Ala Asp Tyr Lys Pro Tyr Leu Glu Glu
    290                 295                 300

GCA GAT GTC CCT GCT GAC TCA AAG AAC AAC ACA TTC ATT GCT GGT GAA    1082
Ala Asp Val Pro Ala Asp Ser Lys Asn Asn Thr Phe Ile Ala Gly Glu
305                 310                 315                 320

TTG CAG TTC GAT TTG CCA CGT TGG GAA GGT GTT CCT TTC TAT GTT CGT    1130
Leu Gln Phe Asp Leu Pro Arg Trp Glu Gly Val Pro Phe Tyr Val Arg
                325                 330                 335

TCA GGT AAG CGT TTG GCT GCC AAG CAA ACA CGT GTT GAT ATT GTA TTT    1178
Ser Gly Lys Arg Leu Ala Ala Lys Gln Thr Arg Val Asp Ile Val Phe
            340                 345                 350

AAG GCT GGC ACA TTC AAC TTT GGT TCA GAA CAA GAA GCA CAA GAA TCA    1226
Lys Ala Gly Thr Phe Asn Phe Gly Ser Glu Gln Glu Ala Gln Glu Ser
        355                 360                 365

GTA CTC TCA ATC ATC ATT GAT CCA AAG GGT GCT ATT GAA TTG AAG CTT    1274
Val Leu Ser Ile Ile Ile Asp Pro Lys Gly Ala Ile Glu Leu Lys Leu
    370                 375                 380

AAC GCT AAG TCA GTT GAA GAT GCC TTC AAC ACC CGC ACA ATC AAC TTG    1322
Asn Ala Lys Ser Val Glu Asp Ala Phe Asn Thr Arg Thr Ile Asn Leu
385                 390                 395                 400
```

```
GAT TGG GCA GTA TCT GAT GAA GAC AAG AAG AAC ACA CCA GAA CCA TAC   1370
Asp Trp Ala Val Ser Asp Glu Asp Lys Lys Asn Thr Pro Glu Pro Tyr
                405                 410                 415

GAA CGT ATG ATT CAC GAT ACA ATG AAT GGT GAC GGA TCA AAC TTT GCT   1418
Glu Arg Met Ile His Asp Thr Met Asn Gly Asp Gly Ser Asn Phe Ala
            420                 425                 430

GAT TGG AAC GGT GTA TCA ATT GCT TGG AAG TTT GTT GAC GCA ATT ACT   1466
Asp Trp Asn Gly Val Ser Ile Ala Trp Lys Phe Val Asp Ala Ile Thr
        435                 440                 445

GCC GTT TAC GAT GCA GAT AAA GCA CCA TTG GAG ACA TAT AAG TCA GGT   1514
Ala Val Tyr Asp Ala Asp Lys Ala Pro Leu Glu Thr Tyr Lys Ser Gly
    450                 455                 460

TCA ATG GGT CCT GAA GCA TCA GAC AAG CTA TTA GCT GAA AAT GGC GAT   1562
Ser Met Gly Pro Glu Ala Ser Asp Lys Leu Leu Ala Glu Asn Gly Asp
465                 470                 475                 480

GCT TGG GTA TTT AAA GGA TAAGCACATT TAAAAAGACC ATCAAACAAA         1610
Ala Trp Val Phe Lys Gly
                485

TCTTTGTTTG ACGGTCTTTT TATATTGTCT GATTTAAGAT GCGTTTGGTT TCACGGAAAA 1670

CGGCTGACAA ATTGGTGTAT TGATCC                                     1696
```

SEQ ID NO:2
ART DER SEQUENZ:Nucleotidsequenz
SEQUENZLÄNGE:72 Basenpaare

STRANGFORM:Einzelstrang

```
RTTTTGAACC ATTTCTTTWC CTAAATAATG ATCAATWCKA AATAATTGRT TATCATCAAA  60

AGCGTTTTCA AA                                                      72
```

### Patentansprüche

1. Glucose-6-Phosphat-Dehydrogenase, **dadurch gekennzeichnet,** daß sie die in SEQ ID NO: 1 dargestellte Aminosäuresequenz enthält.

2. DNA, **dadurch gekennzeichnet,** daß sie den für eine Glucose-6-Phosphat-Dehydrogenase nach Anspruch 1 kodierenden Bereich der in SEQ ID NO:1 dargestellten Nukleinsäure-Sequenz oder eine diesem Bereich im Rahmen der Degeneration des genetischen Codes entsprechende Sequenz enthält.

3. Rekombinanter Vektor nach Anspruch 1, **dadurch gekennzeichnet,** daß er eine oder mehrere Kopien einer DNA nach Anspruch 2 enthält.

4. Plasmid pUC-G6P-DH1.8, wie dargestellt in Abb.1.

5. DNA, **dadurch gekennzeichnet,** daß sie ein nativer Promotor des Glucose-6-Phosphat-Dehydrogenase-Gens ist und die ersten 122 Basen der in SEQ ID NO:1 dargestellten Nukleinsäuresequenz, eine davon abgeleitete Sequenz mit Promotoreigenschaften oder ein Fragment dieses Bereichs mit Promotoreigenschaften enthält.

6. Mikroorganismus,
**dadurch gekennzeichnet,** daß er mit einer DNA nach Anspruch 2 oder einem rekombinanten Vektor nach Anspruch 3 oder 4 transformiert ist.

7. Verfahren zur Gewinnung einer DNA nach Anspruch 2,
**dadurch gekennzeichnet,** daß man
(1) chromosomale Leuconostoc dextranicus (DSM 20187) DNA isoliert und mit einem geeigneten Restriktionsenzym spaltet,
(2) die gespaltene L. dextranicus DNA in einen Vektor einbaut, mit dem Vektor einen geeigneten Wirtsorganismus transformiert und auf diese Weise eine Genbank herstellt,
(3) die Genbank aus (2) mit einer Nukleinsäure-Sonde mustert, die eine für das Glucose-6-Phosphat-Dehydrogenase-Gen spezifische Sequenz besitzt, und
(4) die mit der Sonde aus (3) positiv reagierenden Klone der Genbank analysiert.

8. Verfahren zur Gewinnung eines Proteins nach Anspruch 1,
**dadurch gekennzeichnet,** daß man
(1) einen geeigneten Wirtsorganismus mit einer DNA nach Anspruch 2 oder einem Vektor nach Anspruch 3 oder 4 transformiert,
(2) den transformierten Wirtsorganismus in einem geeigneten Medium kultiviert, und
(3) das Protein aus dem Medium oder den Zellen anreichert.

9. Verfahren zur enzymatischen Bestimmung des Gehalts von Glucose-6-Phosphat in einer Probelösung, wobei man als Bestimmungsenzym Glucose-6-Phosphat-Dehydrogenase verwendet,
**dadurch gekennzeichnet,** daß man eine rekombinante Glucose-6-Phosphat-Dehydrogenase nach Anspruch 1 verwendet.

10. Reagenz zur enzymatischen Bestimmung des Gehalts von Glucose-6-Phosphat in einer Probelösung, wobei man als Bestimmungsenzym Glucose-6-Phosphat-Dehydrogenase verwendet,
**dadurch gekennzeichnet,** daß es eine rekombinante Glucose-6-Phosphat-Dehydrogenase nach Anspruch 1 enthält.

**Claims**

1. Glucose-6-phosphate dehydrogenase,
**wherein**
it contains the amino acid sequence shown in SEQ ID NO:1.

2. DNA,
**wherein**
it contains the region of the nucleic acid sequence shown in SEQ ID NO:1 coding for a glucose-6-phosphate dehydrogenase as claimed in claim 1 or a sequence corresponding to this region within the scope of the degeneracy of the genetic code.

3. Recombinant vector as claimed in claim 1,
**wherein**
it contains one or several copies of a DNA as claimed in claim 2.

4. Plasmid pUC-G6P-DH1.8 as shown in Figure 1.

5. DNA,
**wherein**
it is a native promoter of the glucose-6-phosphate dehydrogenase gene and contains the first 122 bases of the nucleic acid sequence shown in SEQ ID NO:1, a sequence derived therefrom with promoter properties or a fragment of this region with promoter properties.

6. Microorganism,
**wherein**
it is transformed with a DNA as claimed in claim 2 or with a recombinant vector as claimed in claim 3 or 4.

7. Process for the isolation of a DNA as claimed in claim 2,
**wherein**
(1) chromosomalLeuconostoc dextranicus (DSM 20187) DNA is isolated and cleaved with a suitable restriction enzyme,
(2) the cleaved L. dextranicus DNA is incorporated into a vector, a suitable host organism is transformed with the vector and a gene bank is produced in this way,
(3) the gene bank from (2) is screened with a nucleic acid probe which has a sequence specific for the glucose-6-phosphate dehydrogenase gene and
(4) the clones of the gene bank that react positively with the probe from (3) are analyzed.

8. Process for the isolation of a protein as claimed in claim 1,
**wherein**
(1) a suitable host organism is transformed with a DNA as claimed in claim 2 or with a vector as claimed in claim 3 or 4,
(2) the transformed host organism is cultured in a suitable medium and
(3) the protein is purified from the medium or the cells.

9. Method for the enzymatic determination of the content of glucose-6-phosphate in a sample solution in which glucose-6-phosphate dehydrogenase is used as the determination enzyme,
**wherein**
a recombinant glucose-6-phosphate dehydrogenase as claimed in claim 1 is used.

10. Reagent for the enzymatic determination of the content of glucose-6-phosphate in a sample solution in which glucose-6-phosphate dehydrogenase is used as the determination enzyme,
**wherein**
it contains a recombinant glucose-6-phosphate dehydrogenase as claimed in claim 1.

## Revendications

1. Glucose-6-phosphate déshydrogénase caractérisée en ce qu'elle contient la séquence d'acides aminés représentée dans SEQ ID NO: 1.

2. ADN caractérisé en ce qu'il contient le domaine codant une glucose-6-phosphate déshydrogénase selon la revendication 1 de la séquence d'acide nucléique représentée dans SEQ ID NO: 1 ou une séquence correspondant à ce domaine dans le cadre de la dégénérescence du code génétique.

3. Vecteur recombinant selon la revendication 1, caractérisé en ce qu'il contient une ou plusieurs copies d'un ADN selon la revendication 2.

4. Plasmide pUC-G6P-DH1.8 tel que représenté sur la figure 1.

5. ADN caractérisé en ce qu'il s'agit d'un promoteur natif du gène de la glucose-6-phosphate déshydrogénase et en ce qu'il contient les 122 premières bases de la séquence d'acide nucléique représentée dans SEQ ID NO: 1, une séquence à propriétés de promoteur qui en dérive ou un fragment de ce domaine à propriétés de promoteur.

6. Micro-organisme caractérisé en ce qu'il est transformé avec un ADN selon la revendication 2 ou avec un vecteur recombinant selon la revendication 3 ou 4.

7. Procédé d'obtention d'un ADN selon la revendication 2, caractérisé en ce que
(1) on isole l'ADN chromosomique de Leuconostoc dextranicus (DSM 20187) et on le clive avec une enzyme de restriction appropriée,
(2) on introduit dans un vecteur l'ADN de L. dextranicus clivé, on transforme un organisme hôte approprié avec le vecteur et on forme de cette manière une banque génomique,
(3) on crible la banque génomique de (2) avec une sonde d'acide nucléique qui possède une séquence spécifique du gène de la glucose-6-phosphate déshydrogénase, et

(4) on analyse les clones de la banque génomique qui réagissent de manière positive avec la sonde de (3).

**8.** Procédé d'obtention d'une protéine selon la revendication 1, caractérisé en ce que
(1) on transforme un organisme hôte approprié avec un ADN selon la revendication 2 ou avec un vecteur selon la revendication 3 ou 4,
(2) on cultive l'organisme hôte transformé dans un milieu approprié, et
(3) on concentre la protéine à partir du milieu ou des cellules.

**9.** Procédé de détermination enzymatique de la teneur en glucose-6-phosphate d'une solution échantillon, dans lequel on utilise la glucose-6-phosphate déshydrogénase comme enzyme de détermination, caractérisé en ce que l'on utilise une glucose-6-phosphate déshydrogénase recombinante selon la revendication 1.

**10.** Réactif de détermination enzymatique de la teneur en glucose-6-phosphate d'une solution échantillon, dans lequel on utilise la glucose-6-phosphate déshydrogénase comme enzyme de détermination, caractérisé en ce qu'il contient une glucose-6-phosphate déshydrogénase recombinante selon la revendication 1.

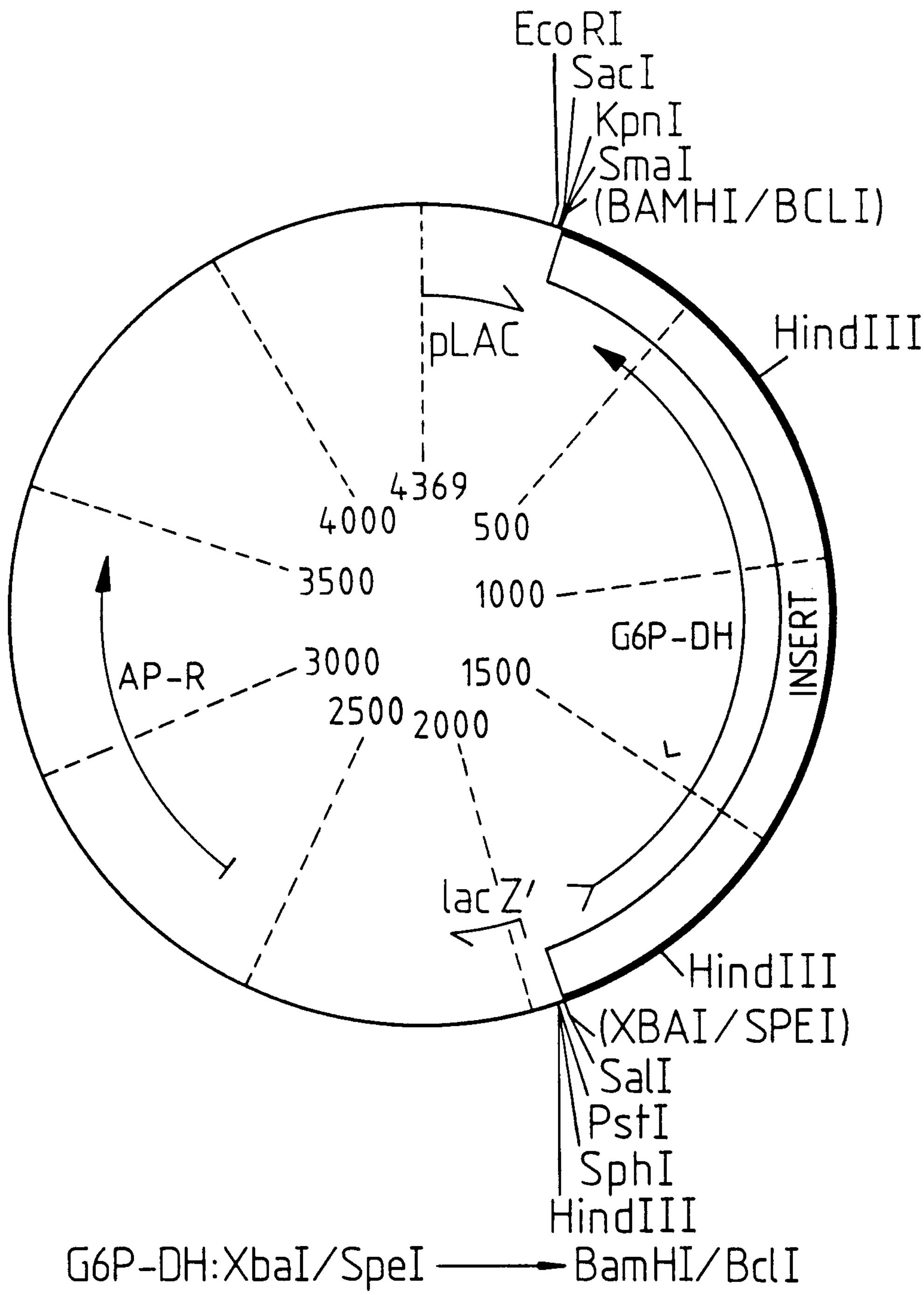
EcoRI
SacI
KpnI
SmaI
(BAMHI/BCLI)
HindIII
pLAC
4369
4000
500
3500
1000
G6P-DH
INSERT
AP-R
3000
1500
2500
2000
lacZ'
HindIII
(XBAI/SPEI)
SalI
PstI
SphI
HindIII
G6P-DH:XbaI/SpeI → BamHI/BclI